Europäisches Patentamt

European Patent Office    (11) Numéro de publication : **0 051 522**

Office européen des brevets    **B1**

(12)    **FASCICULE DE BREVET EUROPÉEN**

(19)

(45) Date de publication du fascicule du brevet :
**12.02.86**

(21) Numéro de dépôt : **81401669.7**

(22) Date de dépôt : **22.10.81**

(51) Int. Cl.⁴ : **C 12 N   7/00, A 61 K 39/205**

(54) **Mutant de septicémie hémorragique virale, vaccin contenant ce mutant et procédé pour sa préparation.**

(30) Priorité : **23.10.80 FR 8022721**

(43) Date de publication de la demande :
**12.05.82 Bulletin 82/19**

(45) Mention de la délivrance du brevet :
**12.02.86 Bulletin 86/07**

(84) Etats contractants désignés :
**BE CH DE GB IT LI NL**

(56) Documents cités :
**FR-A- 2 405 994**
**FR-A- 2 426 737**
**COMPTES RENDUS DE L'ACADEMIE DES SCIENCES
DE PARIS, tome 284, série D, 3 janvier 1977, P. DE
KINKELIN et al. "Isolement d'un Rhabdovinus pathogène de la Truite Fario", pages 101-104**
**COMPTES RENDUS DE L'ACADEMIE DES SCIENCES
DE PARIS, tome 284, série D, 31 janvier 1977, P. DE
KINKELIN et al. "Réaction de la Truite Fario et Arc-
en-Ciel à l'infection par un nouveau Rhabdovirus",
pages 401-404**
**JOURNAL OF VIROLOGY, vol. 36, no. 3, décembre
1980, P. DE KINKELIN et al. "Viral hemorrhagic
septicemia of rainbow trout: selection of a thermoresistant virus variant and comparison of polypeptide
synthesis with the wild-type virus strain", pages 652-
658**

(73) Titulaire : **Etablissement public dit: INSTITUT NATIO-
NAL DE LA RECHERCHE AGRONOMIQUE
149, rue de Grenelle
F-75341 Paris Cedex 07 (FR)**

(72) Inventeur : **De Kinkelin, Pierre
34 Orée de Marly
F-78850 Thiverval Grignon (FR)**
Inventeur : **Le Berre épouse Bearzotti, Monique
49 rue du Petit Pont Montigny le Bretonneux
F-78190 Trappes (FR)**

(74) Mandataire : **Thibon-Littaye, Annick
Cabinet A. THIBON-LITTAYE 11 rue de l'Etang
F-78160 Marly-le-Roi (FR)**

## Description

La présente invention concerne un nouveau vaccin utilisable pour prémunir les salmonidés, et plus particulièrement les truites Arc-en-ciel, contre la Septicémie Hémorragique Virale, ou SHV en abrégé. Elle concerne également sa préparation et un nouveau microorganisme qu'elle fait intervenir.

On sait qu'il existe en fait plusieurs virus responsables de cette maladie, comme il est précisé notamment dans la demande de brevet français déposée le 8 février 1977, sous le n° 77 03438 au nom de la Demanderesse, et son Certificat d'Addition déposé le 19 janvier 1978, sous le n° 78 01556.

Pour prévenir la maladie, on a déjà proposé d'administrer aux poissons, à titre de vaccin, du virus inactivé par traitement chimique. On trouvera dans les demandes de brevets déjà citées tous les détails souhaitables sur un tel vaccin, sa préparation et les conditions de son efficacité.

L'invention propose de remplacer la vaccination connue, qui fait appel à un virus tué, par une vaccination par un virus d'une souche nouvelle, mais vivant, et de permettre ainsi une vaccination plus sûre et plus durable.

Conformément à l'invention, on utilise comme vaccin une forme mutante de virus de SHV qui est pratiquement non virulente dans les conditions de vie habituelles des poissons. Ce vaccin se caractérise en ce qu'il est essentiellement constitué par un mutant de virus de SHV cultivé à température comprise entre 21 et 30 °C et de préférence de l'ordre de 25 °C. Une souche d'un tel mutant a été déposée à la Collection Nationale des cultures de Microorganismes à l'Institut Pasteur, où elle a reçu le n° i-136. L'invention a donc également pour objet un vaccin pour l'immunisation des truites contre la SHV qui se caractérise en ce qu'il est essentiellement constitué par une forme mutante du virus SHV qui est la souche F 25. L'invention a encore pour objet ce microorganisme nouveau caractérisé en ce qu'il est constitué par un virus de SHV sous la forme du mutant déposé à la Collection Nationale de Cultures de Microorganismes. Selon l'invention ce microorganisme utilisable comme vaccin peut être préparé par un procédé caractérisé en ce que l'on cultive du virus de SHV en milieu cellulaire, en effectuant une série de passages à des températures progressivement croissantes depuis une température comprise entre 12 °C et 15 °C jusqu'à une température d'au moins 23 °C, et de préférence comprise entre 23 °C et 28 °C. Le virus de départ se développe bien dans la première gamme de températures, qui est voisine des températures des eaux dans lesquelles vivent les truites. Au contraire, le mutant se développe aux températures nettement plus chaudes de la seconde gamme, mais il a perdu l'essentiel de sa virulence pour les truites. Ce qui est dit ici s'entend naturellement aussi des truites salmonidés sensibles à la SHV.

Dans la mise en œuvre du procédé, chaque passage implique avantageusement d'inoculer la culture en couche monocellulaire par une dose de virus d'environ $10^{-3}$ unité formant plage (UFP) par cellule, de laisser le virus se développer pendant un temps suffisant pour obtenir la destruction complète du tapis cellulaire, généralement de l'ordre de 1 à 5 jours, et de prélever du virus dans le surnageant liquide pour constituer l'inoculum pour le passage suivant. La culture cellulaire est de préférence constituée de cellules de peau de Carpe de la lignée EPC (Epithelioma Papulosum Cyprini), dont un échantillon a fait l'objet d'un dépôt de souche à la Collection Nationale de Cultures de Microorganismes (Institut Pasteur) sous le n° i-039. Le milieu de croissance est généralement du milieu de Stoker, supplémenté par du sérum d'embryon de bovins comme il a été décrit dans les demandes de brevets déjà citées.

Le nombre de passages peut être très variable (généralement de 20 à 150 passages), avec toutefois de préférence au moins 5 passages par gamme de 3 °C d'accroissement de température à partir de 18 °C, ce qui correspond par exemple à au moins 5 passages, et de préférence 10 à 50 passages, à chacun des trois paliers dans l'augmentation progressive de température, à environ 20 °C, 23 °C, et 25-26 °C. C'est ainsi que l'on a obtenu le mutant dénommé F25 (21) qui a fait l'objet d'un dépôt de souche à la même Collection le 1er octobre 1980 sous le n° i-136.

Le virus mutant, ou virus « chaud », se distingue du virus d'origine par sa capacité de multiplication à des températures relativement chaudes auxquelles les virus SHV nocifs à l'égard des truites ne sont pas viables, par exemple de l'ordre de 25 °C.

La forme mutante peut être utilisée à titre de vaccin sur les alevins de truites et autres salmonidés, car elle est à la fois inoffensive mais efficace pour immuniser les poissons contre la contamination par les différents virus de SHV. L'administration peut s'effectuer par toutes voies, et notamment par voie intramusculaire sur chaque individu, à une dose de l'ordre de 10 à $10^4$ UFP, mais on préfère en pratique diluer le virus mutant, à une concentration comprise entre $5 \cdot 10^4$ et $10^6$ UFP/ml, dans l'eau d'un bain de vaccination dans lequel on immerge les alevins pendant un temps suffisant pour leur conférer l'immunisation, généralement de l'ordre de 15 minutes à 3 heures. La température est comprise entre 8 et 15 °C, et de préférence entre 8 et 12 °C, ce qui correspond à la température usuelle des écloseries. Le traitement peut avoir lieu notamment sur des alevins de 1 à 5 g. Le virus est en général produit initialement, en culture cellulaire de poisson et notamment sur cellules EPC avec un titre de $10^7$ à $10^9$ UFP/ml à partir duquel il peut être dilué à toute concentration souhaitée.

On décrira maintenant plus en détails un mode de préparation du vaccin et des essais de vaccination d'alevins de truite Arc-en-ciel en précisant les conditions d'utilisation du vaccin et les résultats qui démontrent son innocuité et son efficacité dans la prévention de la SHV.

## Préparation du vaccin

Le virus SHV de départ est issu de la souche danoise F1. Ce virus a été cultivé en passages successifs sur une culture cellulaire de poisson. La culture cellulaire en monocouche était réalisée en boîte de matière plastique et constituée de cellules de Carpe de la lignée EPC, mises en culture dans du milieu de Stoker, tamponnée à pH 7,4, par du tampon tris -HCL 0,16 M et supplémenté par 10 % de phosphate de tryptose et 10 % de sérum d'embryon de bovin. Après incubation une dizaine d'heures à 28-30 °C, la culture était ramenée à 14 °C pour servir à la croissance du virus.

A chaque passage, le tapis cellulaire était inoculé avec une dose de virus de 0,001 à 0,01 UFP par cellule, on laissait la culture se poursuivre pendant 1 à 3 jours, jusqu'à destruction complète des cellules, et le surnageant infectieux dilué ($10^{-2}$ à $10^{-3}$) constituait l'inoculum du passage suivant. Pour tous les titres indiqués dans ce texte en UFP (Unités Formant Plage, ou « Plaque Forming Units »), le titrage est effectué comme il a été décrit dans les demandes de brevets déjà citées.

Au cours de ces passages successifs on a acclimaté le virus à des températures progressivement croissantes. Après 40 subcultures successives à 20 °C, on a prélevé un clone de la dernière pour inoculer la première d'une série de 20 autres subcultures où la croissance était réalisée chaque fois à 23 °C, pendant 24 heures. Puis l'on a poursuivi les opérations en portant la température à 25,5 °C. Au vingt et unième passage à 25,5 °C, on a obtenu la souche mutante qui a fait l'objet du dépôt déjà mentionné, avec la dénomination F25 (21).

Aux premiers passages à 25,5 °C, comme à chacun des paliers de températures, la croissance du virus devait se poursuivre pendant 3 jours, mais une fois acclimaté, sa capacité de multiplication à cette température est suffisante pour que le temps de croissance soit réduit à 24 heures.

Ce virus est conservé à l'état congelé à − 30 °C ou à − 70 °C. Il peut aussi être lyophilisé.

Le mutant se distingue des virus connus de SHV par le fait qu'il se développe en culture de cellules de poissons à température supérieure à 21 °C et notamment comprise entre 21 °C et 25 °C, alors que les souches qui provoquent la maladie dans les piscicultures ne se développent pas de manière significative à ces températures.

## Essais de vaccination

Pour servir à la vaccination des salmonidés, et plus particulièrement des truites, le virus mutant « chaud » de la souche ci-dessus est remis en culture, par exemple de la manière ci-après :

On inocule la culture de cellules EPC avec un inoculum de virus de 0,01 à 0,001 UFP par cellule. Pour assurer l'adsorption du virus sur les cellules, on maintient une température de 20 °C, pendant une heure, en présence de 50 µg/ml de DEAE Dextran, en s'assurant que le tapis cellulaire soit bien couvert par l'inoculum. De manière à obtenir un titre d'au moins $10^8$ UFP/ml, on laisse le virus se multiplier pendant 24 heures à 25 °C. On le récolte alors par centrifugation (à 3 000 g pendant 15 minutes) et on le congèle à − 30 °C ou à − 70 °C.

Pour une administration individuelle par injection, on recommande une dilution de manière à obtenir des doses de 10 à $10^4$ UFP par poisson sous le volume final convenable à injecter vu la taille de l'animal (de 0,05 à 0,5 ml).

Mais pour une vaccination facile à réaliser dans les conditions d'élevage habituelles en pisciculture, on préfère la vaccination par bain, sur des alevins de 1 à 5 grammes, au moyen d'une suspension aqueuse de virus préparée par dilution de 5 ml de la préparation virale titrant $10^8$ UFP/ml dans 1 litre d'eau. Il suffit d'y plonger les poissons pendant 30 minutes, à la température usuelle des piscicultures soit 8 à 12 °C. Deux semaines après cette vaccination, les poissons résistent aux infections de SHV et cette immunisation persiste au moins pendant 6 mois. Ils sont alors proches de leur taille commerciale.

Dans les essais qui seront rapportés ci-après, on a étudié l'influence de différentes variables, mais sauf indication contraire, les conditions appliquées pour l'administration du vaccin étaient les suivantes :

température du bain : 10 °C

concentration en virus : 5 · $10^5$ UFP/ml

densité des poissons dans le bain : 1 kg par litre

temps de contact : 30 minutes

délai de l'épreuve : 20 jours après l'immunisation

compte des survivants : 4 semaines après l'épreuve

Pour l'épreuve, les poissons vaccinés étaient mis en contact avec du virus SHV de la souche INRA 07-71 telle que déposée à la Collection Nationale de Cultures de Microorganismes (Institut Pasteur), sous le n° i-040, dans les mêmes conditions d'infection par bain que pour la vaccination.

Le pourcentage des survivants dénombrés 4 semaines après l'épreuve est exprimé par rapport à un total de poissons qui comprend les morts intervenues aussi bien pendant la période d'immunisation que pendant la période d'épreuve. Seuls en sont éliminés les poissons accidentés pendant les manipulations.

## Atténuation et pouvoir protecteur

On a administré par bain à des alevins Arc-en-Ciel de 2,5 g, du virus mutant chaud à un premier lot d'alevins, et du virus sauvage de la souche 07-71 à un second lot. On a immergé les alevins pendant 3 heures dans un bain à 10 °C à 50 000 UFP/ml de virus, en proportion de 1 volume de poissons pour 10 volumes de suspension vaccinante. Un troisième lot ne recevait aucun traitement spécial. Quatre semaines après, on a constaté bien sûr une mortalité très importante pour le deuxième lot, mais une mortalité par contre très faible pour le premier lot. On a alors soumis les poissons survivants, ceux du premier et du troisième lots, à une épreuve par le virus sauvage, en leur administrant aux uns et aux autres ce virus, en bain, comme ci-dessus. La mortalité constatée après encore 4 semaines était faible pour le premier lot, correspondant aux poissons vaccinés, très forte au contraire pour le troisième lot, où les poissons n'avaient pas été traités au préalable.

Dans le tableau ci-après des résultats, on a indiqué la proportion des poissons survivants après chaque traitement en pourcentage du nombre des poissons dans le lot soumis au traitement.

| Lot N° | 1 | 2 | 3 |
|---|---|---|---|
| 1ère infection | virus chaud | virus sauvage | 0 |
| % de survie après 28 jours | 94 | 15 | 100 |
| Epreuve | virus sauvage | – | virus sauvage |
| % de survie au 56e jour | 79 | | 11 |

## Durée d'immersion et concentration

L'expérience a porté sur des lots d'une centaine d'alevins chacun, soumis à vaccination en bain, avec des doses variables exprimées par la concentration du bain en vaccin, variant de $5 \cdot 10^4$ à $5 \cdot 10^5$ UFP/ml. La durée du contact avec la solution vaccinante variait de 15 à 150 minutes. L'épreuve avait lieu 21 jours après la vaccination. On a obtenu les résultats du tableau ci-après.

| Vaccination | | | | | |
|---|---|---|---|---|---|
| Dose UFP/ml | 0 | $2.10^5$ | $5.10^5$ | $2.10^5$ | $5.10^4$ |
| Durée minutes (témoin) | | 30 | 15 | 15 | 150 |
| Total de poissons | 101 | 97 | 98 | 99 | 95 |
| Morts | 2 | 7 | 7 | 4 | 2 |
| Epreuve | | | | | |
| Total poissons | 99 | 88 | 91 | 94 | 93 |
| Morts | 75 | 27 | 21 | 35 | 28 |
| Survivants | 23 | 61 | 70 | 59 | 65 |
| Bilan % survivants | 22,8 | 62,9 | 71,4 | 59,6 | 68,4 |

## Essais d'innocuité

Le virus vaccin, réisolé de poissons morts pendant la période d'immunisation, ne montre aucune augmentation de virulence par rapport au vaccin initial.

4

**0 051 522**

Ainsi, sur des lots de 33 alevins de 2,5 g ayant respectivement reçu différents virus, les % de mortalité au bout de 4 semaines étaient les suivants :

| Virus | % morts |
|---|---|
| 0 | 3 |
| virus pathogène | 87,8 |
| virus vaccin | 6 |
| virus vaccin réisolé | 9 |

De même dans une autre expérience effectuée sur des animaux d'une autre origine (160 à 148 par lot)

| Virus | % morts |
|---|---|
| 0 | 0,6 |
| virus vaccin | 15,7 |
| virus vaccin réisolé | 13 |

De même encore, au cours de passages successifs de poisson à poisson, effectués à 6 jours d'intervalle par injection, le virus n'a plus été réisolé à partir du 7e passage. Donc, au lieu de retrouver de la virulence, il a eu le comportement inverse en disparaissant.

Enfin, le virus chaud cultivé de nouveau à froid (14 °C) sur cellules pendant 5 passages consécutifs ne montre pas d'augmentation de virulence :

| Virus | % morts |
|---|---|
| 0 | 10 |
| virus vaccin (5 passages à 14 °C) | 27 |
| virus vaccin normal | 25 |

D'autre part, on s'est préoccupé de savoir si les animaux vaccinés excrétaient un virus qui pouvait être dangereux pour les poissons situés en aval. Dans une première expérience, aucune mortalité significative n'est survenue chez les sujets recevant l'eau contenant des vaccinés et les 3 morts recueillis (sur 100) ont conduit à des résultats d'examens virologiques négatifs. Donc le virus vaccin ne semble pas dangereux pour les poissons situés en aval, mais il est cependant excrété à faible dose car les survivants précédents, confrontés ensuite au virus pathogène, ont montré une résistance significative.

| Animaux | % de mortalité |
|---|---|
| Témoins non vaccinés | 60,1 |
| Témoins ayant reçu l'eau des vaccinés | 40,4 |
| Vaccinés | 27 |

La confirmation d'une légère excrétion du virus vaccin est apparue dans les essais d'innocuité par injection au cours desquels le virus a pu être réisolé de 1 sur 12 des animaux marqués et ajoutés dans l'aquarium contenant les poissons vaccinés 24 heures après l'injection à ces derniers. Mais aucune mortalité n'est survenue au cours de ces transmission par contact.

L'innocuité a aussi été vérifiée en fonction de la température. Alors que dans la nature, la contamination des truites par la SHV ne se produit guère qu'à des températures inférieures à 15 °C, on a réalisé la vaccination dans les conditions, pour le reste, standard, en maintenant la température de bain à 18 °C.

On a obtenu les résultats suivants :

(Voir tableau page 6)

5

|  | à 10°C | | à 18°C | |
|---|---|---|---|---|
| Vaccination | oui | non | oui | non |
| Total initial | 101 | 198[+] | 78 | 86 |
| Morts | 31 | 15 | 9 | 4 |
| Disparus | 0 | 4 | 0 | 0 |
| Epreuve (à10°C) | | | | |
| Total initial | 70 | 179 | 69 | 82 |
| Morts | 6 | 129 | 14 | 59 |
| Disparus | 0 | 3 | 3 | 2 |
| Survivants | 61 | 47 | 52 | 21 |
| Bilan | | | | |
| % Survivants | 60 | 23,7 | 66,6 | 24,4 |

(+) en 2 aquariums

### Contamination par différents virus SHV

Le vaccin protège les truites contre différents types de SHV. Après vaccination par 50 000 UFP/ml de virus mutant pendant 180 minutes à 10 °C sur des alevins de 2,5 g, l'épreuve a été réalisée 31 jours après, avec soit du virus SHV 1 de la souche 07-71, soit du virus 23-75 de la souche INRA qui a été déposée à la Collection Nationale de Cultures de Microorganismes (Institut Pasteur) sous le n° i-042. Ce dernier virus est particulièrement virulent. Dans chaque cas la comparaison a été faite avec un lot témoin d'alevins non vaccinés.

| Virus d'épreuve : | 07-71 | | 23-75 | |
|---|---|---|---|---|
| Vaccination : | oui | non | oui | non |
| Total initial | 150 | 149 | 150 | 150 |
| Morts | 9 | 3 | 15 | 3 |
| Disparus | 2 | 0 | 2 | 0 |
| Epreuve : | | | | |
| Total initial | 139 | 146 | 133 | 147-22[+] |
| Morts | 60 | 106 | 75 | 123 |
| Survivants | 79 | 40 | 58 | 2 |
| Bilan : | | | | |
| % Survivants | 52,7 | 26,9 | 38,7 | 1,6 |

(+) Animaux tués ou tombés sur le sol au cours des manipulations. Le pourcentage des survivants est calculé par rapport au total (survivants + morts en période d'immunisation et en période d'épreuve).

### Administration par injection

Le vaccin a été injecté par voie intramusculaire, à la dose de 10-20 UFP par individu. L'épreuve a eu lieu au moyen de virus 07-71 dans les conditions standard 31 jours après la vaccination. La comparaison avec un lot témoin de poissons non vaccinés a fait apparaître les résultats ci-après.

| Vaccination | oui | non |
|---|---|---|
| Total initial | 50 | - |
| Morts | 10 | - |
| | | |
| Epreuve : | | |
| Infectés | 40 | 50 |
| Morts | 4 | 40 |
| Survivants | 36 | 10 |
| % Survivants | 72 | 20 |

Recherche du virus chez les animaux vaccinés

Sur des truitelles vaccinées de 2,2 g dans les conditions standard définies ci-dessus, on a recherché la présence du virus 3, 7, 15 et 21 jours après la vaccination. Le virus n'a jamais pu être mis en évidence sur plus de 50 % des animaux. Dans des lots de 100 poissons, il a été trouvé sur 20 % d'entre eux à 3 jours, sur 15 % à 7 jours, sur aucun à 20 jours.

Essai à grande échelle

En procédant dans les conditions standard définies ci-dessus (5 · $10^5$ UFP/ml, 30 minutes), 5 000 poissons de 2 g ont été vaccinés en laboratoire et 5 000 témoins ont été traités de la même façon avec du milieu de culture cellulaire.

Au bout de 14 jours, 300 vaccinés et 300 témoins ont été répartis en aquarium (75 poissons par aquarium) pour être soumis à l'épreuve virulente au laboratoire.

Le reste des animaux a été transféré en pisciculture le même jour pour y subir l'épreuve virulente naturelle.

Le 20e jour après l'immunisation, la mortalité respective enregistrée dans les deux lots était de 7 % chez les vaccinés et de 2 % chez les témoins.

L'épreuve de laboratoire, effectuée le 20e jour, s'est traduite au bout de 4 semaines par une survie de 70 % des animaux vaccinés contre 20 % des témoins. Des résultats identiques ont été obtenus avec la contamination directe des poissons (virus ajouté dans l'eau) ou par contamination au moyen de poissons précontaminés (contact).

L'épreuve de pisciculture a conduit à une mortalité débutant 30 jours après l'introduction des poissons et a laissé subsister 80 % des vaccinés et 40 % des témoins. Dans ce cas, le plus grand espace dont disposaient les poissons peut rendre compte du relatif ralentissement de la contagion.

Les deux épreuves ont donc révélé l'efficacité de la protection conférée par le vaccin même administré à des effectifs 30 à 50 fois plus élevés que ceux des lots expérimentaux habituels.

**Revendications**

1. Nouveau microorganisme constitué par un virus de SHV (Septicémie Hémorragique Virale de la truite), sous la forme du mutant déposé à la Collection Nationale de Cultures de Microorganismes à l'Institut Pasteur le 1er octobre 1980 sous le n° i-136.

2. Vaccin pour l'immunisation des truites contre la SHV, caractérisé en ce qu'il est essentiellement constitué par une forme mutante du virus SHV cultivée à température comprise entre 21 et 30 °C, et de préférence de l'ordre de 25 °C.

3. Vaccin pour l'immunisation des truites contre la SHV, caractérisé en ce qu'il est essentiellement constitué par une forme mutante du virus SHV, qui est la souche F 25 (21) telle que déposée à la Collection Nationale de Cultures de Microorganismes à l'Institut Pasteur le 1er octobre 1980 sous le n° i-136.

4. Vaccin selon la revendication 3, caractérisé en ce que ladite forme mutante est produite en culture cellulaire de poisson, notamment sur cellules Epithelioma Papulosum Cyprini.

5. Vaccin selon la revendication 4, caractérisé en ce que la forme mutante est produite à un titre de l'ordre de $10^7$ à $10^9$ UFP/ml.

6. Vaccin selon les revendications 2 à 5, caractérisé en ce que ladite forme mutante est contenue en concentration comprise entre 10 et $10^4$ UFP par dose unitaire de 0,05 à 0,5 ml, dans un véhicule sous forme fluide convenant pour être administré en injections.

7. Vaccin selon la revendication 2 ou 3, caractérisé en ce que ladite forme mutante est contenue en concentration comprise entre $5 \cdot 10^4$ et $10^6$ UFP/ml. dans un bain aqueux pour l'immersion d'alevins de truite.

8. Procédé de préparation d'un vaccin pour l'immunisation des truites contre la SHV selon la revendication 2, caractérisé en ce que l'on cultive du virus de SHV en milieu cellulaire en effectuant une série de passages à des températures progressivement croissantes depuis une température comprise entre 12 °C et 15 °C jusqu'à une température d'au moins 23 °C, et de préférence entre 23 °C et 28 °C.

9. Procédé selon la revendication 8, caractérisé en ce que l'on effectue au moins 5 passages par gamme de 3 °C d'accroissement de température à partir de 18 °C, avec de préférence au total 20 à 150 passages de 18 °C à 25-26 °C.

## Claims

1. Novel microorganism constituted by a virus of VHS (Viral Hemorrhagic Septicemia of trout) in the form of the mutant registered in the National Collection of Cultures of Microorganisms at the Pasteur Institute on October 1st, 1980 under n° i-136.

2. Vaccine for immunization of trout against VHS, characterized in that it is essentially constituted by a mutant form of the VHS virus cultivated at a temperature comprised between 21 and 30 °C and preferably of the order of 25 °C.

3. Vaccine for immunization of trout against VHS, characterized in that it is essentially constituted by a mutant form of the VHS virus which is the strain F 25 (21) as registered in the National Collection of Cultures of Microorganisms at the Pasteur Institute on October 1st, 1980 n° i-136.

4. Vaccine in accordance with claim 3, characterized in that said mutant from is produced in a fish cell culture, especially on cells of Epithelioma Papulosum Cyprini.

5. Vaccine in accordance with claim 4, characterized in that the mutant form is produced with a titer of the order of $10^7$ to $10^9$ UFP/ml.

6. Vaccine in accordance with claims 2 to 5, characterized in that said mutant form is contained in a concentration comprised between 10 and $10^4$ UFP per unitary dose of 0.05 to 0.5 ml in a vehicle in fluid form which is suitable for administration in the form of injections.

7. Vaccine in accordance with claim 2 or 3, characterized in that said mutant form is contained in a concentration comprised between $5 \times 10^4$ and $10^6$ UFP/ml in an aqueous bath for the immersion of young trout.

8. Method of preparation of a vaccine for immunization of trout against VHS in accordance with claim 2, characterized in that VHS virus is cultivated in a cell medium by carrying out a series of passes at progressively increasing temperatures from a temperature comprised between 12 °C and 15 °C and a temperature of at least 23 °C and preferably between 23 °C and 28 °C.

9. Method in accordance with claim 8, characterized in that at least five passes are carried out per range of 3 °C of temperature rise from 18 °C with preferably a total of 20 to 150 passes from 18 °C to 25-26 °C.

## Patentansprüche

1. Neuer Microorganismus, bestehend aus einem Virus der SHV (Septicémie Hémorragique Virale = Virus-Blutvergiftung) der Forelle in Form des Mutanten, der bei der Collection Nationale de Cultures de Microorganismes beim Institut Pasteur am 1. Oktober 1980 unter der Nummer i-136 niedergelegt worden ist.

2. Impfstoff zur Immunisierung von Forellen gegen SHV, dadurch gekennzeichnet, daß er vornehmlich aus einer mutierten Form des SHV-Virus besteht, die bei einer Temperatur zwischen 21 und 30 °C, vorzugsweise bei etwa 25 °C, kultiviert wird.

3. Impfstoff zur Immunisierung von Forellen gegen SHV, dadurch gekennzeichnet, daß er im wesentlichen aus einer mutierten Form des SHV-Virus, Stamm F 25 (21), besteht, wie bei der Collection Nationale de Cultures de Microorganismes beim Institut Pasteur am 1. Oktober 1980 unter der Nummer i-136 niedergelegt.

4. Impfstoff gemäß Anspruch 3, dadurch gekennzeichnet, daß die erwähnte mutierte Form des SHV-Virus aus einer Fischzellenkultur entwickelt wird, hauptsächlich aus den Zellen des Epithelioma Papulosum Cyprini.

5. Impfstoff gemäß Anspruch 4, dadurch gekennzeichnet, daß die mutierte Form in einer Titer der Größenordnung $10^7$ - $10^9$ PFU/ml (Plaque Forming Units = Fleckenbildende Einheit) hergestellt ist.

6. Impfstoff gemäß den Ansprüchen 2-5, dadurch gekennzeichnet, daß die erwähnte mutierte Form in einer Konzentration zwischen 10 und $10^4$ PFU in einer Einheitsdosis von 0,05-0,5 ml einer für Injektionen geeigneten Aufnahmeflüssigkeit vorliegt.

7. Impfstoff gemäß den Ansprüchen 2 und 3, dadurch gekennzeichnet, daß die erwähnte mutierte Form in einer Konzentration zwischen $5 \cdot 10^4$ und $10^6$ PFU in einem Wasserbad zum Tauchen der Forellenbrut enthalten ist.

8. Verfahren zur Herstellung eines Impfstoffes zur Immunisierung von Forellen gegen SHV gemäß Anspruch 2, dadurch gekennzeichnet, daß der SHV-Virus in einem Zellenmilieu kultiviert wird, wobei er eine Reihe von ansteigenden Temperaturen durchläuft, die bei einer Temperatur zwischen 12 °C und 15 °C beginnen und bis zu einer Temperatur von wenigstens 23 °C, vorzugsweise zwischen 23 °C und 28 °C, aufhören.

9. Verfahren gemäß Anspruch 8, dadurch gekennzeichnet, daß wenigstens 5 Passagen mit Temperaturabständen von jeweils 3 °C durchlaufen werden, beginnend mit 18 °C, wobei vorzugsweise insgesamt 20-150 Passagen von 18 °C bis 25-26 °C durchlaufen werden.